# EUROPEAN PATENT APPLICATION

(11) **EP 3 222 312 A1**
(43) Date of publication of application: **27.09.2017**
(21) Application number: 17162055.2
(22) Date of filing: 21.03.2017
(51) Int. Cl.: A61M 5/315, A61M 5/31

(54) **UNCOATED PISTON / CYLINDER SYSTEMS WITH LOW OPERATION FORCE AND REDUCED STICK / SLIP**

(30) Priority: 21.03.2016 EP 16161489
(71) Applicant: BA.Evolution GmbH, 49479 Ibbenbüren (DE)
(72) Inventor: Mirtschin, Steffen, 16552 Schildow (DE)
(74) Representative: Hoeger, Stellrecht & Partner Patentanwälte mbB

(57) **Abstract**

The invention relates to a cylinder-piston system comprising a cylinder, wherein at least an inner section of the inner surface of the cylinder is formed from a first polymer material (C), a piston (1), comprising at least one sealing section on the outer surface (14) of the piston (1), which is formed from a second polymer material (P), wherein the cylinder and the piston (1) are positioned or positionable in such a way that during a sliding movement of the piston (1) with respect to the cylinder the inner section of the cylinder and the sealing section of the piston (1) slide on each other. One of said polymer materials, particularly the first polymer material (C), comprises at least a polar polymer material, particularly is selected from a polar polymer material, and the other polymer material, particularly the second polymer material (P), comprises at least a nonpolar polymer material, particularly is selected from a nonpolar polymer material.

## Description

The invention relates to a cylinder-piston system, particularly for use in injection syringes, for medical, cosmetic, or fitness applications, diffusors, insulin pens, perfusors, inhalators, hydraulic systems, or pneumatic systems.

Cylinder-piston systems are widely used for example in medical technology, cosmetics and the beauty industry, as well as in the life science industry and industrial technology. In cylinder-piston systems, a piston is moved in a cylinder in order to displace and/or transport a medium, particularly a liquid. For example, medical injection syringes are commonly applied to inject pharmaceutical compounds into a patient body. Common applications of disposable or reusable medical injection syringes include intravitreal injection syringes in ophthalmology, diffusors, particularly for artificial nutrition, insulin pens for insulin injection in diabetes patients, syringes for botulinum toxin injections in medical cosmetics, syringes for hyaluronic acid injections in medicine, particularly sports medicine and fitness applications, as well as surgical devices for application of pharmaceutical agents in a patient body.

In most applications, the interface between the cylinder and the piston has to be well sealed from the exterior in order to avoid leakage of the displaced/ transported fluid. Tight sealing is especially important in medical injection syringes, particularly in order to maintain sterility of the injected composition, or to prevent leakage of damaging, particularly toxic substances, for example during injection of botulinum toxin, radio-chemicals, or artificial feeding.

Sealing can be technically realized by an interference fit (also known as press fit or friction fit) of the piston in the cylinder. Therein, particularly the piston or a part of the piston has a slightly greater diameter than the corresponding cavity of the cylinder. Alternatively or additionally, seals, gaskets, and/or O-rings can be used to tightly seal a cylinder-piston system from the exterior.

As a result of the tight sealing, the static friction (also known as adhesive friction) between the cylinder and the piston can exceed the dynamic friction (also known as sliding friction) between the cylinder and the piston. This property leads to high movement forces as well as the so-called stick-slip effect, which is characterized by a sudden sliding movement of the piston in the cylinder when an external force is applied to the piston. Particularly in medical injection syringes, the stick-slip effect is disadvantageous, because the abrupt pressure increase of the injected fluid may cause irritation and/or injury in the patient, particularly in intravitreal injections in ophthalmology.

In order to reduce the stick-slip effect, while maintaining a tight sealing against the exterior, elastic syringe systems are known from the prior art. These elastic syringe systems utilize external lubricants, such as silicon oil or other external lubricants, which are commonly applied to the inside of the cylinder and/or the outside of the piston. Especially in medical applications, such lubricants are disadvantageous as the application process of the external lubricants does not assure 100% accuracy with regards to applied volume and application position. The major disadvantage results from the fact that external lubricants have a higher risk to become at least partially dissolved or suspended in the transported fluid, and may thus be mixed with the pharmaceutical compounds injected into the patient. This may result in unwanted side effects, such as toxicity, allergies, or inactivation of the compound. In all current cases where external lubricants were used, fractions of the used lubricants remaining inside the human body were detected.

Therefore, the objective of the present invention is to provide a cylinder-piston system, which is characterized by a reduced stick-slip effect in the absence of external lubricants, while maintaining a tight sealing of the cylinder-piston system to the exterior. The described functions are assured by using internal lubricants, which are part of any polymer processing material inherently making the polymer material flow in designated processing conditions like injection or extrusion.

This objective is attained by the subject matter of the independent claim 1. Embodiments of the invention are claimed by the dependent claims 2 to 19, and are further described below.

According to a first aspect of the invention, a cylinder-piston system is provided. The cylinder-piston system comprises a cylinder, wherein at least an inner section of the inner surface of the cylinder is formed from a first polymer material, and a piston, comprising at least one sealing section on the outer surface of the piston, which is formed from a second polymer material. Therein, the cylinder and the piston are positioned or positionable in such a way that during a sliding movement of the piston with respect to the cylinder the inner section of the cylinder and the sealing section of the piston slide on each other, and wherein one of the polymer materials, particularly the first polymer material, comprises at least a polar polymer material, particularly is selected from a polar polymer material and the other polymer material, particularly the second polymer material, comprises at least a nonpolar polymer material, particularly is selected from a nonpolar polymer material.

Surprisingly it was found that an interface between a polar polymer material and a nonpolar polymer material markedly reduces the stick-slip effect of cylinder-piston system without the need for external lubricants. According to the state of the art, these external lubricants are applied to both piston and cylinder. In the present invention, the stick-slip effect could be dramatically reduced to only half of what is achieved today with external lubrication systems by keeping the same operating force compared to the externally lubricated systems.

In the scope of the present specification, the term polymer designates a macromolecule, which is comprised of a plurality of subunits.

In the scope of the present specification, the term polymer material designates a material comprising a polymer or a mixture of polymers.

In the scope of the present specification, the term polar designates a property of a molecule of having a permanent electric dipole moment. That is, the electron density of a polar molecule is distributed such that a partial charge is localized at or near a specific atom of the molecule in the absence of an external electric field due to the different electronegativity of the atoms.

In the scope of the present specification, the term non-polar designates a molecule having essentially no permanent electric dipole moment. That is the electron density of a non-polar molecule is distributed such that essentially no partial charge is localized at specific atoms of the molecule in the absence of an external electric field.

In certain embodiments, the complete inner surface of the cylinder is formed from the first polymer material. In certain embodiments, the entire cylinder, is formed from the first polymer material. In certain embodiments, the inner surface of the cylinder is coated with the first polymer material.

In certain embodiments, a partial inner surface of the cylinder is formed from the first polymer material. In certain embodiments, a partial inner surface of the cylinder is coated with the first polymer material.

In certain embodiments, the partial inner surface is a sliding surface, particularly a maximum sliding surface, of the cylinder. Therein, the sliding surface is the partial surface, which is contacted by at least a part of the piston during a downstroke or upstroke of the piston.

Therein, downstroke of the piston refers to a movement of the piston in a direction, such that the volume of the cylinder containing or adapted to contain the medium becomes smaller, and upstroke of the piston refers to a movement of the piston in a direction, such that the volume of the cylinder containing or adapted to contain the medium becomes larger, which allows the creation of a vacuum to allow the filling of this lager volume with e.g. liquids.

In certain embodiments, the sliding surface corresponds to a medium surface. There in the medium surface is the part of the inner surface of the cylinder, which contacts the medium when the cylinder of the assembled cylinder-piston system is filled to its intended or maximum volume.

In certain embodiments, the complete outer surface of the piston is formed from the second polymer material. In certain embodiments, the entire piston is formed from the second polymer material. In certain embodiments, the piston is coated with the second polymer material.

In certain embodiments, a partial outer surface of the piston is formed from the second polymer material, wherein particularly the partial outer surface contacts the sliding surface of the cylinder during a downstroke or upstroke of the piston, more particularly while sealing against the outside environment.

In certain embodiments, the piston is assembled from at least two parts, wherein at least one of the at least two parts is formed from the second polymer material.

In certain embodiments, the piston comprises a sealing means, particularly a seal, gasket, or O-ring, which is adapted to seal the cylinder-piston system against leakage of medium to the exterior of the cylinder-piston system, wherein the sealing means is formed from the second polymer material.

The first polymer material preferably comprises or is selected from at least a polar polymer material. Preferably, the interface energy with respect to water of the polar polymer material is between 30 mN/m and 60 mN/m, particularly between 35 mN/m and 50 mN/m, more particularly between 40 mN/m and 45 mN/m.

The second polymer material preferably comprises or is selected from at least a nonpolar polymer material. Preferably, the interface energy with respect to water of the nonpolar polymer material is less than 25 mN/m, and is particularly between 10 mN/m and 20 mN/m.

In certain embodiments, the difference of interface energies with respect to water between the first polymer material and the second polymer material is at least 10 mN/m, in particular at least 18 mN/m, more particularly at least 25 mN/m. In particular, the interface energy with respect to water of the first polymer material is higher than the interface energy with respect to water of the second polymer material.

In the scope of the present specification, the term interface energy is defined as the energy that has to be spent in order to establish an interface between two phases, particularly a polar substance and a non-polar substance.

An interface between a polar and a non-polar surface is characterized by greatly reduced molecular interactions compared to polar/polar and non-polar/non-polar interfaces. In a cylinder-piston system this effect results in superior sliding properties in the absence of external lubricants, such as silicon oil. External lubricants have adverse effects in medical applications, particularly because the external lubricants may be dissolved in the medium and be injected in the patient. Thus, in particular, the cylinder-piston system according to the invention, which does not utilize external lubricants, allows the construction of biocompatible injection syringes or other medical devices without any effect on serum or medication composition, particularly according to IOS 10993-5, ISO 10993-10, USP <88>, and ISO 10993-4 standards. In addition, the combination of polar and non-polar polymers in this invention also allows long term storage without significant adhesion between the polar and non-polar polymers.

In particular, the interface energy of a surface can be measured at the relevant surfaces under the relevant conditions by applying different test ink samples onto the surface and assessing wettability two seconds after application of the respective test ink, such as designated by DIN 53364/ ISO 8296.

Interaction energies of different polymer were tested using test ink samples having known interaction energies from 10 to 72 mN/m in steps of 2 mN/m. Test ink samples were purchased from Softal Electronic GmbH (Hamburg, Germany). Therein, the respective test ink was applied to the sliding surface of syringe cylinders made from different materials using a paintbrush. Dependent on the difference in interaction energy between the respective test ink and the respective polymer material, droplets formed on the surface after different times. The measured interaction energy of the respective polymer material is the interaction energy of the respective test ink, which had not yet formed visible droplets 2 seconds after application onto the surface. Table 1 shows a range of interaction energies measured by the described method.

**Table 1. Interaction energies of different polymer materials**

| | Interaction energy σ_{S} [mN/m] | Polarity in relation to TPE polymer |
|---|---|---|
| **TPE** | 10...20 | base |
| **PP/PE/COC** | 28....32 | low |
| **PMMA** | 35...40 | medium...high |
| **PETG / PBT** | 38...45 | high |
| **PETG** (**Skygreen S2008)** | 42 | high |

In certain embodiments, the first polymer material comprises a ball indentation hardness according to ISO 2039-1 in the range of 130 to 160 MPA.

A sufficient hardness of the cylinder ensures stability and breakage resistance of the cylinder-piston system.

In certain embodiments, the second polymer material comprises a hardness of Shore A in the range of 33 to 80, in particular of 40 to 55.

In certain embodiments, the first polymer material comprises a ball indentation hardness according to ISO 2039-1 in the range of 130 to 160 MPA, and the second polymer material comprises a hardness of Shore A in the range of 33 to 80, in particular of 40 to 55.

A combination of a rigid, transparent cylinder with a softer piston results in enhanced sealing properties both during downstroke and upstroke of the piston. This also allows the construction of gastight cylinder-piston systems. A tight sealing also ensures that the interior of the cylinder-piston system remains sterile during use, which is especially important for medical applications.

For example, PET-G comprises a ball indentation hardness according to ISO 2039-1 in the range of 130 to 160 MPA, and TPE comprises a hardness of Shore A in the range of 33 to 80.

In certain embodiments, the compression set A of the second polymer material is in the range of 0 to 30 %, in particular in the range of in the range of 0 to 20 %, measured at a temperature of 60° C. Therein, the term "compression set A" refers to a compression set measured at constant force.

A high resilience, corresponding to a low compression set, in the range of 0 to 30 %, of the softer second polymer material influences sealing of the cylinder-piston system.

In certain embodiments, the first polymer material comprises an amorphous structure with a degree of crystallisation of less than 5 %.

In certain embodiments, the first polymer material comprises a super-amorphous structure with a degree of crystallisation of less than 3 %.

In certain embodiments, the first polymer material comprises an amorphous structure near the relevant inner sliding surface with a degree of crystallisation of less than 5 %.

In certain embodiments, the first polymer material comprises a super-amorphous structure near the relevant inner sliding surface with a degree of crystallisation of less than 3 %.

In certain embodiments, the first polymer material comprises an amorphous structure with a degree of crystallisation of less than 5 % in a surface layer - with respect to the inner wall of the cylinder - with a depth of up to 0.1, in particular up to 0.05 mm.

In certain embodiments, the first polymer material comprises an amorphous structure with a degree of crystallisation of less than 3 % in a surface layer - with respect to the inner wall of the cylinder - with a depth of up to 0.1, in particular up to 0.05 mm.

In certain embodiments, the second polymer material comprises an amorphous structure with a degree of crystallisation of less than 10 %, particularly less than 5 %, more particularly less than 3%.

Surprisingly, it was found that a super-amorphous structure of the first polymer material and/or the second polymer material improves the sliding properties and the stick-slip behaviour of a piston when sliding on a cylinder surface comprising the second polymer material.

Without wishing to be bound by theory, it is assumed that this effect is due to improved migration of internal lubricants from the interior of the respective polymer material to its surface by means of the amorphous structure.

Furthermore, the material properties of the first material benefit from this super-amorphous structure as the haptic near and at the sliding surface becomes an uninterrupted even area with only minimum interruptions and unevenness within its fine structures.

It has to be noted that the super-amorphous structure of the first material also improves the migration of the internal lubricants. However, due to the reduced amount in the first polymer material the effect is not as strong as discussed above for the second material.

Therefore, the effect is improved by using both materials in a cylinder-piston system.

In certain embodiments, the first polymer material comprises internal lubricants.

In certain embodiments, the second polymer material comprises internal lubricants.

In certain embodiments, the first polymer material and the second polymer material comprise internal lubricants.

In particular, the internal lubricants comprise or are selected from at least one of the group of acid amides, more particularly primary acid amides, most particularly erucamide, oleamide, or stearamide, secondary amides, most particularly ethylene bis stearamide (EBS) or ethylene bis oleamide (EBO), acid esters, more particularly poly ethylene mono stearate (PEMS), poly ethylene di stearate (PEDS), poly ethylene tri stearate (PETS), poly ethylene co-acrylic acid stearate (PEAS), glycerol monostearate (GMS), glycerol monooleate (GMO), Montan Wax, stearyl stearate, or distearyl phthalate, fatty acids, more particularly saturated fatty acids, most particularly lauric acid, myristic acid, palmitic acid, or stearic acid, or unsaturated fatty acids, most particularly oleic acid or erucic acid, hydrocarbon waxes, more particularly polyethylene, polypropylene, Oxidized Polyethylene Wax (OPE), or paraffin, metallic soaps, more particularly calcium stearate, magnesium stearate, lead stearate, aluminium stearate, sodium stearate, tin stearate, barium stearate, cobalt stearate, or zinc stearate, or polytetrafluorethylen.

In certain embodiments, the internal lubricants comprise, particularly are selected from at least one of the group of paraffin, wax, polypropylene, polytetrafluorethylen or zinc sterate.

In the scope of the present specification, the term internal lubricant designates a substance, which is included in the manufacturing process of polymer material, and which results in favourable flow properties for injection or extrusion processes.

Such internal lubricants are incorporated within the molecular structure of the polymer material, and can diffuse or migrate to the surface of the polymer material in small amounts, where they bind oxygen molecules. When this surface is exposed to an aqueous solution, only a small amount of internal lubricant is dissolved in the aqueous solution. However, the presence of internal lubricants at the surface of the polymer material reduces static friction of the polymer material at an interface with a second polymer material. This effect improves the sliding properties and stick-slip behaviour of a cylinder-piston system according to the present invention utilizing such an interface between two polymer materials, especially when combined with an amorphous structure of the polymer material at the sliding inner surface of the cylinder/ piston meeting point.

An amorphous structure of the polymer material containing the internal lubricants improves the diffusion of internal lubricants to the surface and thereby results in greatly improved sliding properties and stick-slip behaviour at an interface with a second polymer material.

Compared to a polymer surface coated with external lubricants such as silicon oil, a much smaller amount of internal lubricants is dissolved in an aqueous solution in contact with the polymer material.

In order to analyze the stick-slip properties of different polymer materials, force to distance measurements were performed using conventional BD U 100 syringes (Table 2) and syringes based on a 0.5 ml cylinder-piston system according to the present invention (Table 3).

Samples 47 to 49 are replicate measurements using the same type of BD U 100 syringes. These conventional syringes have a cylinder made of polypropylene and a piston stopper made of nitrile butadien rubber (NBR), and comprise silicon oil as an external lubricant.

Samples 63 to 66 are replicate measurements of syringes using a cylinder-piston system according to the invention (Skygreen S2008 for the cylinder and ProvaMed®3150 for the piston). The syringes were equipped with a 30 gauge needle using water as a medium and a velocity of 60 mm/min at room temperature filled with boiled water at room temperature. The time [s] indicated in the tables 2 and 3 is the time after which the piston reaches its final position.

**Table 2. Stick-slip properties of conventional syringes (with external lubrication)**

| Sample No. | Maximum force [N] | Time [s] |
|---|---|---|
| 47 | 1.39 | 39.42 |
| 48 | 1.23 | 39.39 |
| 49 | 1.41 | 39.30 |

**Table 3. Stick-slip properties of syringes using the cylinder-piston system according to the invention**

| Sample No. | Maximum force [N] | Time [s] |
|---|---|---|
| 63 | 0.740 | 39.41 |
| 64 | 0.898 | 39.41 |
| 65 | 0.900 | 39.42 |
| 66 | 0.687 | 39.42 |

For a structural analysis of PET polymer materials, scanning electron microscopy experiments were performed. Therein, a first PETG S2008 test cylinder injection molded at a temperature below 18 °C, and a second PETG S2008 control test cylinder injection molded at 28 °C were cut using a microtome, and sputtered for scanning electron microscopy. A second sample of each cylinder was cut using a microtome, and the samples were etched using an etchant containing chromic acid and subjected to scanning electron microscopy (SEM). To ensure that the test cylinder injection molded below 18 °C indeed had the intended sliding properties compared to the control sample, sliding force measurements were performed with both samples. As expected, a sliding force below 1 N was measured for the test cylinder injection molded below 18 °C, indicating good sliding properties, whereas for the control sample a sliding force above 2 N was measured.

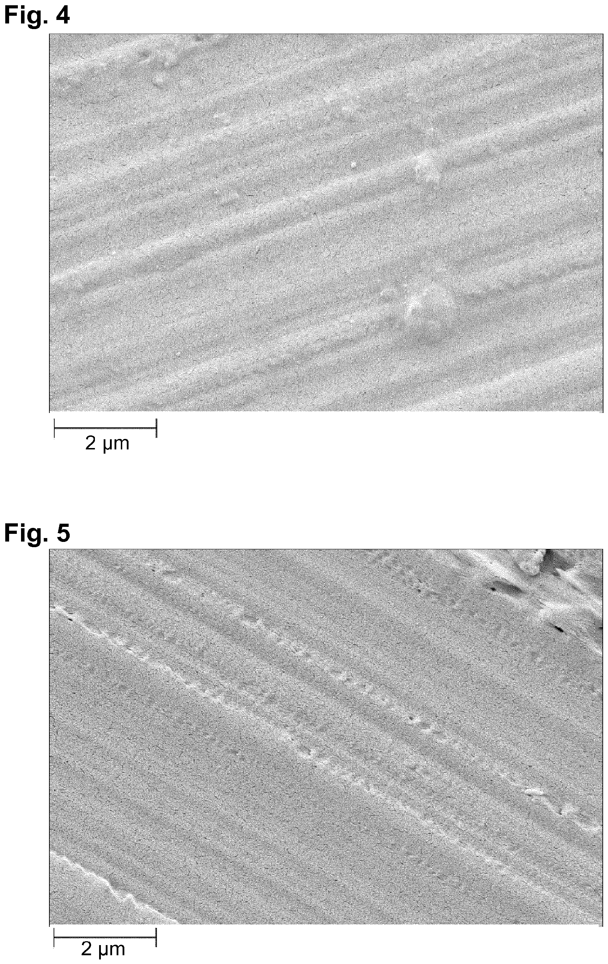

The SEM images of sections indicate a more planar structure of the sample injection molded below 18 °C (Fig. 5) compared to the control sample (Fig. 4), suggesting a different structure and configuration of the samples with less superordinate structures. The depicted homogeneous amorphous configuration with only very few crystalline structures (Fig. 5) in the boundary area, which is located in the vicinity of the sliding interface of a cylinder-piston system according to the invention indicates a super-amorphous structure.

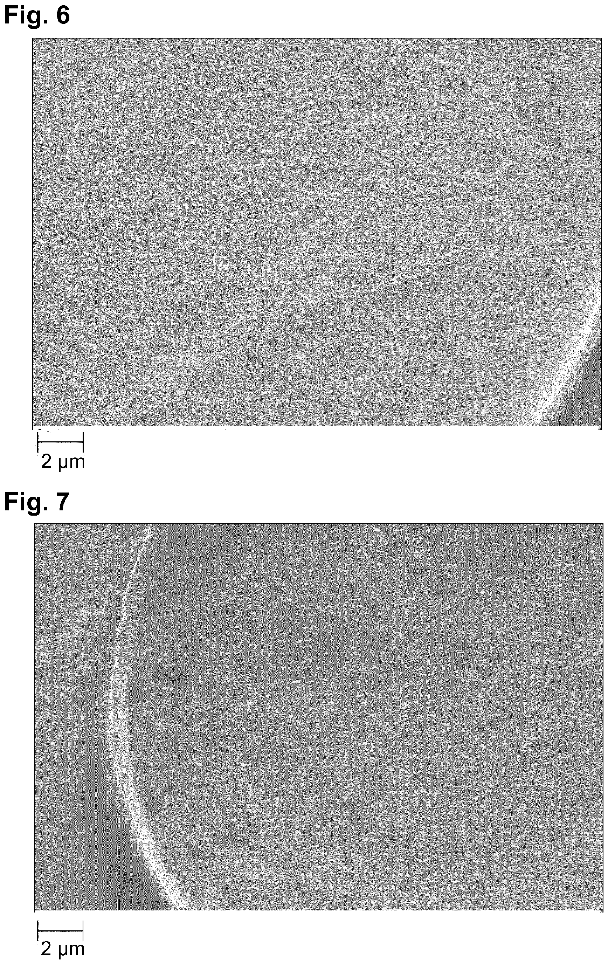

The etched samples show superordinate structures in more detail (Fig.6, Fig. 7). These images indicate that the displayed boundary areas in the vicinity of the sliding interface possess less superordinate structures in the sample injection molded below 18 °C compared to the control sample (Fig. 6, Fig. 7). These superordinate structures are mostly located further from the boundary area in the sample injection molded below 18 °C (Fig. 7).

Without wishing to be bound by theory, it is assumed that the superordinate structures are the result of spherulites comprising amorphous and crystalline sectors.

Typical lamellar structures of the crystallites within the spherulites could not be detected, probably due to non-optimal etching conditions (Fig. 6, Fig. 7).

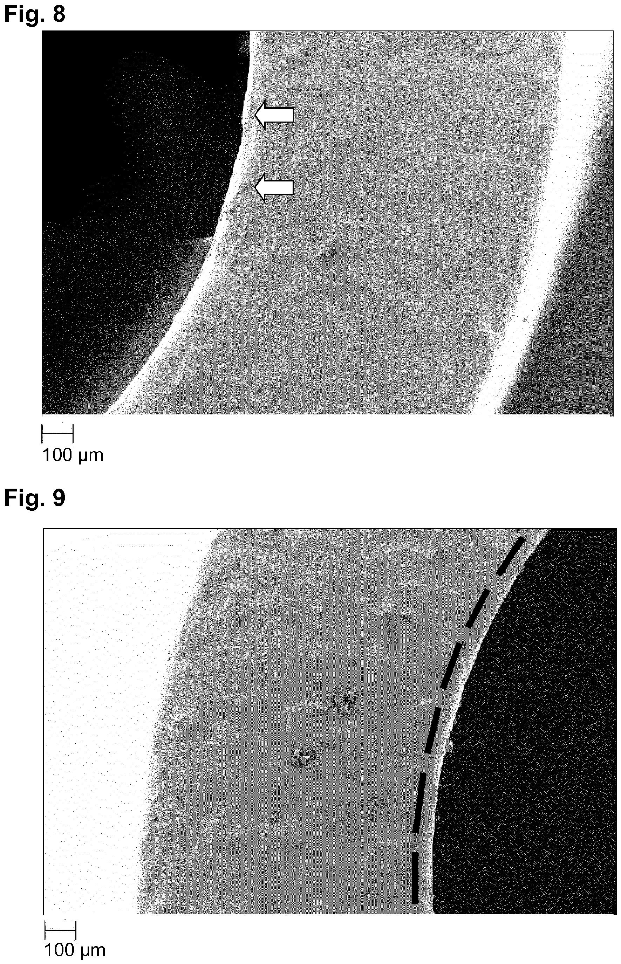

Fig. 8 and 9 depict lower magnification images (150-fold magnification) of the sample injection molded below 18 °C (Fig. 9) and the control sample (Fig. 8). Here, the boundary area of the cylinder, which is in contact with the piston sliding along this wall area near the visible (black dashed line in Fig. 9). Whereas the control sample shows crystalline structures in the boundary area (Fig. 8, arrows), these structures are missing in the boundary area of the sample injection molded at 18 °C (Fig. 9).

The SEM images of the etched samples depicted in Fig. 8 and 9 were analyzed using image processing software. Therein, clearly visible superordinate structures, which were completely positioned in the field of view, were overlaid with a polygon by manual operation. The pixels within a respective polygon were counted by the image processing program. The length of a pixel was determined to approximately 0.56 µm according to a measurement of the scale bar.

Table 4 shows SEM results of the sample cooled to below 18 °C during injection moulding. Therein, a total of 115 spherulite structures were analyzed, and a mean spherulite area of 48.4 µm² was determined with a standard deviation of 2.1 µm².

**Table 4. Etching SEM measurement of PETG injection molded below 18 °C**

| **Sample** | **Total area [µm²]** | **Mean spherulite area [µ**m**²]** | **No. of spherulites** |
|---|---|---|---|
| 1 | 14600 | 49.1 | 21 |
| 2 | 13500 | 48.7 | 11 |
| 3 | 41400 | 52.8 | 11 |
| 4 | 12300 | 47.6 | 16 |
| 5 | 18500 | 46.9 | 15 |
| 6 | 22600 | 47.0 | 16 |
| 7 | 15500 | 46.1 | 10 |
| 8 | 13200 | 48.8 | 15 |
| **Mean** | **19000** | **48.4** | **14.4** |

**Table 5. Etching SEM measurement of PETG injection molded at 28 °C**

| **Sample** | **Total area [µm²]** | **Mean spherulite area [µm²]** | **No. of spherulites** |
|---|---|---|---|
| 1 | 23400 | 58.8 | 10 |
| 2 | 16100 | 59.7 | 14 |
| 3 | 17000 | 60.5 | 14 |
| 4 | 12200 | 62.3 | 23 |
| 5 | 17200 | 62.7 | 17 |
| 6 | 14700 | 57.4 | 10 |
| 7 | 16000 | 54.9 | 10 |
| 8 | 14000 | 53.9 | 9 |
| **Mean** | **16300** | **58.8** | **13.4** |

Table 5 shows SEM results of the control sample injection molded at 28 °C. 107 spherulites of this material were analyzed by SEM, and a mean spherulite area of 58.8 µm² was determined with a standard deviation of 3.2 µm².

In certain embodiments, the first polymer material comprises, particularly is selected from a transparent material, in particular Polyethylenterephthalate (PET), Polybutylenterephthalate (PBT), Polymethylmethacrylate (PMMA), polysulfone (PSU), Polycarbonate (PC), Polystyrene (PS), Cyclo-Olefin-Copolymeres (COC), Polypropylene (PP) or Polyethylene (PE).

In certain embodiments, the first polymer material comprises, particularly is selected from Polyethylenterephthalate (PET), Polybutylenterephthalate (PBT), Polymethylmethacrylate (PMMA), polysulfone (PSU), Cyclo-Olefin-Copolymeres (COC), Polypropylene (PP) or Polyethylene (PE), in particular Polyethylenterephthalate (PET), Polybutylenterephthalate (PBT), Polymethylmethacrylate (PMMA) or polysulfone (PSU), more particularly Polyethylenterephthalate (PET), Polybutylenterephthalate (PBT) or polysulfone (PSU).

Transparent, optically clear cylinder materials allow the application of the cylinder-piston system in an injection syringe, allowing visible control of the injection process by the operator. In addition, a cylinder made from the described materials comprises favorable fracture strength properties, in particular impact strength tested according to ASTM D3763 and ASTM D4812 standards.

In certain embodiments, the second polymer material comprises, particularly is selected from a thermoplastic elastomer (TPE).

In the scope of the present specification, the term thermoplastic elastomer designates a material having elastic mechanical properties, wherein the material becomes pliable or moldable upon heating and solidifies upon cooling. An example of a thermoplastic elastomer is described in the document US 2015/0005436 A1, paragraph [0018-0019].

Thermoplastic elastomers which can be used as the second polymer material according to the invention are commercially available und the name "ProvaMed". The TPE named "ProvaMed®3150" used herein was purchased from ACTEGA GmbH (Wesel, Germany).

Thermoplastic elastomer materials comprise favorable hardness, resilience properties, and are non-polar, resulting in improved sliding and stick-slip properties, while maintaining good sealing properties of the cylinder-piston system.

In certain embodiments, the thermoplastic elastomer comprises, particularly is selected from a hydrogenated styrenic block copolymer, particularly styrene-ethylene/butylene-styrene (SEBS), styrene-ethylene/propylene-3-methylbutene-styrene (SEPS), styrene-ethylene-ethylene/propylene-styrene (SEEPS), or styrene-isoprene-styrene (SIPS).

In certain embodiments, the thermoplastic elastomer (TPE) is a urethane based thermoplastic elastomer (TPU), or a derivate thereof.

In certain embodiments, the first polymer material comprises, particularly is selected from a transparent material, in particular Polyethylenterephthalate (PET), Polybutylenterephthalate (PBT), Polymethylmethacrylate (PMMA), polysulfone (PSU), Polycarbonate (PC), Polystyrene (PS), Cyclo-Olefin-Copolymeres (COC), Polypropylene (PP) or Polyethylene (PE), and the second polymer material (P) comprises, particularly is selected from a thermoplastic elastomer (TPE).

The described first polymer materials are polar and the described second polymer materials are non-polar, such that their combination may be applied in a favorable cylinder-piston system with improved stick-slip behavior by similar operating force (compared with externally lubricated systems).

In certain embodiments, the first polymer material comprises, particularly is selected from Polyethylenterephthalate (PET), Polybutylenterephthalate (PBT), Polymethylmethacrylate (PMMA), polysulfone (PSU), Cyclo-Olefin-Copolymeres (COC), Polypropylene (PP), Polyethylene (PE), in particular Polyethylenterephthalate (PET), Polybutylenterephthalate (PBT), Polymethylmethacrylate (PMMA), or polysulfone (PSU), more particularly Polyethylenterephthalate (PET), Polybutylenterephthalate (PBT), or polysulfone (PSU).

In certain embodiments, the first polymer material comprises, particularly is selected from Polyethylenterephthalate (PET), Polybutylenterephthalate (PBT), Polymethylmethacrylate (PMMA), polysulfone (PSU), Cyclo-Olefin-Copolymeres (COC), Polypropylene (PP), Polyethylene (PE), in particular Polyethylenterephthalate (PET), Polybutylenterephthalate (PBT), Polymethylmethacrylate (PMMA) or polysulfone (PSU), more particularly Polyethylenterephthalate (PET), Polybutylenterephthalate (PBT), or polysulfone (PSU), and the second polymer material (P) comprises, particularly is selected from a thermoplastic elastomer (TPE).

In certain embodiments, the first polymer is selected from Polyethylenterephthalate (PET) or polysulfon (PSU).

In certain embodiments, the first polymer is selected from Polyethylenterephthalate (PET) or polysulfon (PSU), and the second polymer is selected from a thermoplastic elastomer (TPE).

In the scope of the present specification, the term polysulfon (PSU) refers to a polymer having the structure shown in formula 1.

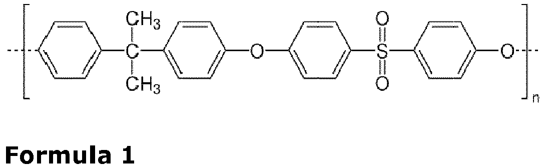

In certain embodiments, the first polymer is selected from Polyethylenterephthalate (PET).

In certain embodiments, the Polyethylenterephthalate (PET) comprises, particularly is selected from Polyethylenterephthalate-Glycol (PETG) or Polyethylenterephthalate-Cyclohexane Dimethanol (PET-CHDM).

In the scope of the present specification, the term Polyethylenterephthalate-Glycol (PETG) refers to a glycol-modified Polyethylenterephthalate (PET) having the structure shown in formula 2.

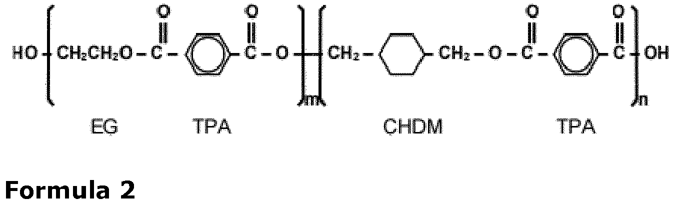

In the scope of the present specification, the term Polyethylenterephthalate-Cyclohexane Dimethanol (PET-CHDM) refers to a Polyethylenterephthalate (PET), in which at least 0.1 % of ethylene glycol is replaced by Cyclohexane Dimethanol (CHDM). The PET-CHDM used herein was purchased from SK Chemicals Co., Ltd.

In particular, Polyethylenterephthalate-Cyclohexane Dimethanol (PET-CHDM) shown as formula 1 is commercially available under the name "Skygreen". It is a transparent amorphous copolyester produced by the reaction of terephthalic acid (TPA) with ethylene glycol (EG) in which a certain amount of the ethylene glycol is replaced with cyclohexane dimethanol (CHDM). The addition of CHDM prevents crystallisation, leading to improved processability combined with outstanding toughness, clarity and chemical resistance.

PETG is available under the commercial name "Skygreen S2008". The PETG used herein was purchased from SK Chemicals Co., Ltd.

Such replacement of subunits of PET reduces the tendency of the PET material to crystallize, allowing to provide an amorphous or super-amorphous material at particular areas.

In certain embodiments, the first polymer material comprises, particularly is selected from Polyethylenterephthalate (PET) and the second polymer material comprises, particularly is selected from a thermoplastic elastomer (TPE).

In certain embodiments, the first polymer material comprises, particularly is selected from Polyethylenterephthalate-Glycol (PETG) or Polyethylenterephthalate-Cyclohexane Dimethanol (PET-CHDM) and the second polymer material comprises, particularly is selected from a thermoplastic elastomer.

Both materials can be sterilized, particularly by ethylene oxide treatment, or gamma irradiation without loss of their favorable mechanical properties. Furthermore, both materials are stable during storage for up to 6 months at temperatures between 0 °C and 40 °C. During ethylene oxide sterilization at 60 °C, the materials are stable for at least 40 hours. Moreover, both materials are characterized by a low tendency to creep and a high dimensional stability. Both materials are also available as pure materials adapted for use in medicine technology.

In certain embodiments, the sealing section of the piston is adapted to press or presses against the inner surface of the cylinder of the cylinder-piston system, wherein the ratio (Ep/Ec) between the maximum piston extension (Ep) of the piston with respect to the longitudinal axis of the cylinder-piston system and the corresponding maximum cavity extension of the cavity defined by the cylinder is 1.02 to 1.10.

That is, the cylinder is a hollow cylinder comprising a cavity. Here, the longitudinal axis of the cylinder-piston-system is positioned in the direction of movement of the piston in the cylinder.

The corresponding extensions are to be measured in the same angle with respect to the longitudinal axis of the cylinder, wherein the extension of the sealing section (Ep) is measured when the sealing section is not pressed by the cylinder.

In certain embodiments, the cylinder and the piston have corresponding circular cross-sections.

If in an embodiment of the invention the cylinder and the piston have corresponding circular cross-sections, the diameter of the sealing section is 1.02 to 1.10 times larger than the diameter of the cavity of the cylinder so that there is a permanent preload applied onto the sealing section in order to realize a tight sealing on the inner surface of the cylinder.

In certain embodiments, the piston of the cylinder-piston-system comprises a first sealing section and a second sealing section as well as a recess section, wherein the recess section is provided along the longitudinal axis of the cylinder-piston-system between the first sealing section and the second sealing section, and wherein the maximum extension of the recess section is smaller than the maximum extension of the first sealing section and of the second sealing section, respectively.

That is, the recess section is adapted to absorb pressure applied onto the piston when the piston is pushed into the cylinder and against a medium provided in the cavity of the cylinder. If the cylinder and the piston have corresponding circular cross sections, the diameter of the recess section is smaller than the diameters of the first and second sealing sections.

Even during pushing the piston into the cylinder there is enough space between the surface of the recess section and the inner surface of the cylinder in order to avoid contact between the surfaces of these elements, so that a stick-slip phenomenon between the recess section and the inner surface of the cylinder will not occur. That is, the surface of the recess section may be made of a different material than the first and second materials.

In certain embodiments, the first sealing section and/or the second sealing section is/ are an integral part of a body of the piston.

In certain embodiments, the first sealing section and/or the second sealing section is/ are an additional element/ additional elements provided on the body of the piston.

If the first sealing section and/or the second sealing section is/ are an integral part of a body of the piston, the body has a corresponding circumferential protrusion, such as a piston ring or a circumferential sealing lip. Furthermore, the piston may comprise an opening adapted to fix a piston rod in a form fit and/or a force fit.

In certain embodiments, the first sealing section is provided on a proximal side of the piston and/or the second sealing section is provided on a distal side of the piston, wherein the cross section of the first sealing section along the longitudinal axis of the cylinder-piston-system has a convex shape on its outer surface, and the cross section of second sealing section along the longitudinal axis of the cylinder-piston-system has a linear shape on its outer surface.

In certain embodiments, a conical section, which is positioned between the recess section and the second sealing section, may be provided, wherein the diameter of the bigger end of the conical section is equal to the diameter of the second sealing section.

In certain embodiments, a conical protrusion may be positioned on the proximal side of the first sealing section wherein the conical protrusion is adapted to move into a complementary formed space in the cylinder for the removal of residues of the cylinder.

According to a second aspect of the invention, a method for introducing or depositing a fluid in or on a medium is provided. In the method, a cylinder-piston system according to the first aspect of the invention is provided, wherein the fluid is introduced in the cylinder of the cylinder-piston system and is deployed by a movement of the piston from the cylinder introducing or depositing of fluid in or on a medium.

The cylinder of the cylinder-piston system of the described architecture may comprise a first polymer material according to the embodiments described above, and/or the piston of the cylinder-piston system of the described architecture may comprise a second polymer material according to the embodiments described above. The combination of the described architecture and the polar and non-polar materials forming an interface further improves the stick-slip properties while maintaining the operating force at low level of the cylinder-piston system.

According to a third aspect of the invention, a use of the cylinder-piston system according to the first aspect of the invention for introducing or depositing a fluid in or on a medium is provided.

In certain embodiments, the cylinder-piston system is used in an injection syringe, particularly a medical injection syringe.

In certain embodiments, the cylinder-piston system is used for medical injections, particularly ophthalmological injections, insulin injections, or hyaluron injections.

In certain embodiments, the cylinder-piston system is used for ophthalmological injections, particularly intravitreal injections.

Good sliding properties and a reduction of the stick-slip effect is especially important during ophthalmological injections, because an abrupt pressure increase of the injected fluid may cause pain and/or injury in the patient.

In a preferred embodiment of the invention, (i) the fill volume of the cylinder is in the range from 0.35 ml to 1.0 ml, in particular from 0.5 ml to 0.85 ml, and/or (ii) the inner diameter of the cylinder is in the range from 3 mm to 4 mm, and/or (iii) the length of the cylinder along which the piston can slide is in the range from 35 mm to 80 mm, in particular from 50 mm to 65 mm. Cylinder-piston systems which such dimension are particularly preferred for ophtalmological injections, but may also be useful in other fields of application.

In certain embodiments, the cylinder-piston system is used in a diffusor, particularly for artificial nutrition.

In certain embodiments, the cylinder-piston system is used as an insulin pen, particularly for use in insulin injection.

In certain embodiments, the cylinder-piston system is used for cosmetic injection, particularly for injection of botulinum toxin.

In certain embodiments, the cylinder-piston system is used for injections in fitness or sports medicine, particularly for hyaluron injections.

In certain embodiments, the cylinder-piston system is used as part of a medical instrumentation, in particular a piston cylinder comprising sealings which are equipped with superior sliding properties in medical instrumentation.

In certain embodiments, the cylinder-piston system is used as part of a surgical instrument, particularly for applying pharmacological compounds into a patient body.

In certain embodiments, the cylinder-piston system is used as a perfusor.

In certain embodiments, the cylinder-piston system is used as part of an inhalator.

In certain embodiments, the cylinder-piston system is used as part of a hydraulic system.

In certain embodiments, the cylinder-piston system is used as part of a pneumatic system.

According to a fourth aspect of the invention, a method for manufacturing a cylinder-piston system according to the first aspect of the invention is provided. The method comprises providing a first polymer material and a second polymer material, wherein the first polymer material comprises, particularly is selected from a polar polymer material and the second polymer material comprises, particularly is selected from a nonpolar polymer material, and manufacturing at least an inner section of the inner surface of a cylinder derived from the first polymer material, and manufacturing at least one sealing section on the outer surface of a piston derived from the second polymer material.

According to a fifth aspect of the invention, a method for manufacturing a cylinder of a cylinder-piston system according to the first aspect of the invention is provided. The method comprises a plastification step, in which a polymer material is plastified, an injection step, in which the plastified polymer material is injected into a casting mould, and a solidification step, in which the polymer material is solidified.

In certain embodiments, the plastification step is performed by heating of the polymer material.

In certain embodiments, the plastification is performed by extruding of the polymer material.

In certain embodiments, the solidification step is performed by cooling of the polymer material.

In certain embodiments, the polymer material is cooled to a temperature below 22, in particular 18 °C prior to the injection step.

A temperature below 18 °C during injection results in an amorphous structure near and at the sliding inner cylinder surface, which has favourable effects on sliding properties and stick-slip behaviour of the cylinder-piston system.

According to a sixth aspect of the invention, a method for manufacturing a piston of a cylinder-piston system according to the first aspect of the invention is provided. The method comprises a plastification step, in which a polymer material is plastified, an injection step, in which the plastified polymer material is injected into a casting mould, and a solidification step, in which the polymer material is solidified.

In certain embodiments, the plastification step is performed by heating of the polymer material.

In certain embodiments, the solidification step is performed by cooling of the polymer material.

In certain embodiments, the polymer material is cooled to a temperature below 22 °C, particularly 20 °C, prior to the injection step.

A temperature below 22 °C, in particular 20 °C, during injection results in an amorphous structure, which has favourable effects on sliding properties and stick-slip behaviour of the cylinder-piston system.

According to a seventh aspect of the invention, a polymer material comprising an amorphous or super-amorphous structure with a degree of crystallisation of less than 5 %, particularly less than 3 %, is provided.

Surprisingly, it was found that a super-amorphous structure of the first polymer material and/or the second polymer material improves the sliding properties and the stick-slip behaviour of a piston when sliding on a cylinder surface comprising the second polymer material. Without wishing to be bound by theory, it is assumed that this effect is due to improved migration of internal lubricants from the interior of the respective polymer material to its surface by means of the amorphous structure on the second material.

The material properties of the first material benefit from this super amorphus structure as the haptic near and at the sliding surface becomes an uninterrupted even area with only minimum interruptions and unevenness within its fine structures. This benefit is believed to occur at two occasions, the manufacturing process -demolding from the forming core- and during use in conjunction with the second material.

In certain embodiments, the polymer material is a polar polymer material. In particular, the interface energy with respect to water of the polymer material is between 30 mN/m and 60 mN/m, particularly between 35 mN/m and 50 mN/m, more particularly between 40 mN/m and 45 mN/m.

It was found that an interface between a polar polymer material and a nonpolar polymer material markedly reduces the stick-slip effect of cylinder-piston system without the need for external lubricants. In particular, the stick-slip effect could be dramatically reduced to less than 0.5 N compared to 1.0 N measured for comparable systems of the state of the art.

An interface between a polar and a non-polar surface is characterized by greatly reduced molecular interactions compared to polar/ polar and non-polar/ non-polar interfaces. In a cylinder-piston system this effect results in superior sliding properties in the absence of external lubricants, such as silicon oil. External lubricants have adverse effects in medical applications. In addition, the combination of polar and non-polar polymers in this invention also allows long term storage without significant adhesion between the polar and non-polar polymers.

In certain embodiments, the polymer material comprises a ball indentation hardness according to ISO 2039-1 in the range of 130 to 160 MPA.

A sufficient hardness of the polymer ensures stability and breakage resistance of objects manufactured from the polymer.

In certain embodiments, the polymer material comprises internal lubricants.

Such internal lubricants are incorporated within the molecular structure of the polymer material, and can diffuse to the surface of the polymer material in small amounts, where they bind oxygen molecules. When this surface is exposed to an aqueous solution, only a small amount of internal lubricant is dissolved in the aqueous solution. However, the presence of internal lubricants at the surface of the polymer material reduces static friction of the polymer material at an interface with a second polymer material. This effect improves the sliding properties and stick-slip behaviour of a cylinder-piston system according to the present invention utilizing such an interface between two polymer materials, especially when combined with an amorphous structure of the polymer material.

An amorphous structure of the polymer material containing the internal lubricants improves the diffusion of internal lubricants to the surface and thereby results in greatly improved sliding properties and stick-slip behaviour at an interface with a second polymer material.

Compared to a polymer surface coated with external lubricants such as silicon oil, a much smaller amount of internal lubricants is dissolved in an aqueous solution in contact with the polymer material.

In certain embodiments, the polymer material comprises, particularly is selected from a transparent material, in particular Polyethylenterephthalate (PET), Polybutylenterephthalate (PBT), Polymethylmethacrylate (PMMA), polysulfone (PSU), Polycarbonate (PC), Polystyrene (PS), Cyclo-Olefin-Copolymeres (COC), Polypropylene (PP) or Polyethylene (PE).

In certain embodiments, the polymer material comprises, particularly is selected from Polyethylenterephthalate (PET), Polybutylenterephthalate (PBT), Polymethylmethacrylate (PMMA), polysulfone (PSU), Cyclo-Olefin-Copolymeres (COC), Polypropylene (PP), Polyethylene (PE), in particular Polyethylenterephthalate (PET), Polybutylenterephthalate (PBT), Polymethylmethacrylate (PMMA) or polysulfone (PSU), more particularly Polyethylenterephthalate (PET) or Polybutylenterephthalate (PBT) or polysulfone (PSU).

In certain embodiments, the polymer material comprises, particularly is selected from Polyethylenterephthalate (PET) or polysulfone (PSU).

In certain embodiments, the polymer material comprises, particularly is selected from Polyethylenterephthalate (PET). In other words, the term "particularly is selected from" is to be understood as "particularly consists of" Polyethylenterephthalate (PET).

In certain embodiments, the Polyethylenterephthalate (PET) comprises, particularly is selected from Polyethylenterephthalate-Glycol (PETG) or Polyethylenterephthalate-Cyclohexane Dimethanol (PET-CHDM).

In certain embodiments, the polymer material comprises, particularly is selected from Polyethylenterephthalate-Glycol (PETG) or Polyethylenterephthalate-Cyclohexane Dimethanol (PET-CHDM).

In certain embodiments, the polymer material is conditioned at a temperature below 18 °C during molding.

A super-amorphous structure, especially near the material surface, resulting in good sliding and stick-slip properties, has been shown for PET derivatives.

According to an eighth aspect of the invention, a method for manufacturing a polymer material according to the seventh aspect of the invention is provided. The method comprises providing a polymer material, particularly comprising or selected from Polyethylenterephthalate (PET), Polybutylenterephthalate (PBT), Polymethylmethacrylate (PMMA), polysulfone (PSU), Polycarbonate (PC), Polystyrene (PS), Cyclo-Olefin-Copolymeres (COC), Polypropylene (PP), Polyethylene (PE), plastifying the polymer material, particularly by heating, conditioning the polymer material by subjecting the polymer material to a temperature of below 22 °C, in particular of below 18 °C, and solidifying the material, particularly by cooling.

In certain embodiments, the polymer material is cooled in a single step.

In certain embodiments, the polymer material is injected into an injection mould during conditioning.

A conditioning step at a temperature below 22 °C, in particular of below 18 °C, has been shown to result in a super-amorphous structure of the polymer material with a low degree of crystallisation.

In certain embodiments, the polymer material is a polar polymer material. In particular, the interface energy with respect to water of the polymer material is between 30 mN/m and 60 mN/m, particularly between 35 mN/m and 50 mN/m, more particularly between 40 mN/m and 45 mN/m.

In certain embodiments, the polymer material comprises internal lubricants.

In certain embodiments, the polymer material comprises, particularly is selected from a transparent material, in particular Polyethylenterephthalate (PET), Polybutylenterephthalate (PBT), Polymethylmethacrylate (PMMA), polysulfone (PSU), Polycarbonate (PC), Polystyrene (PS), Cyclo-Olefin-Copolymeres (COC), Polypropylene (PP) or Polyethylene (PE).

In certain embodiments, the polymer material comprises, particularly is selected from Polyethylenterephthalate (PET), Polybutylenterephthalate (PBT), Polymethylmethacrylate (PMMA), polysulfone (PSU), Cyclo-Olefin-Copolymeres (COC), Polypropylene (PP), Polyethylene (PE), in particular Polyethylenterephthalate (PET), Polybutylenterephthalate (PBT), Polymethylmethacrylate (PMMA), or polysulfone (PSU), more particularly Polyethylenterephthalate (PET), Polybutylenterephthalate (PBT), or polysulfone (PSU).

In certain embodiments, the Polyethylenterephthalate (PET) comprises, particularly is selected from Polyethylenterephthalate-Glycol (PETG) or Polyethylenterephthalate-Cyclohexane Dimethanol (PET-CHDM).

In certain embodiments, the polymer material comprises, particularly is selected from Polyethylenterephthalate (PET).

In certain embodiments, the polymer material comprises, particularly is selected from Polyethylenterephthalate-Glycol (PETG) or Polyethylenterephthalate-Cyclohexane Dimethanol (PET-CHDM).

In certain embodiments, the polymer material comprises, particularly is selected from polysulfone (PSU).

### Short description of the figures

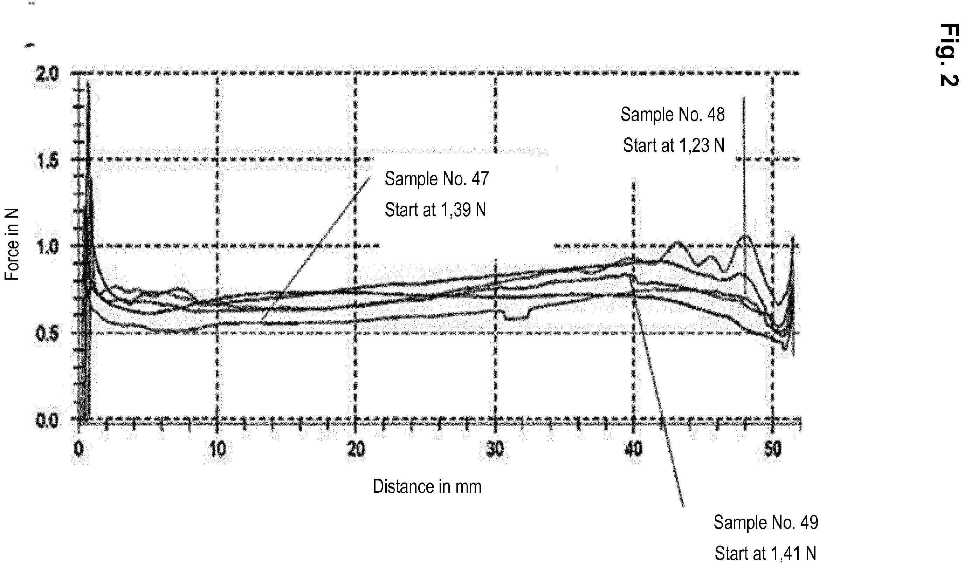

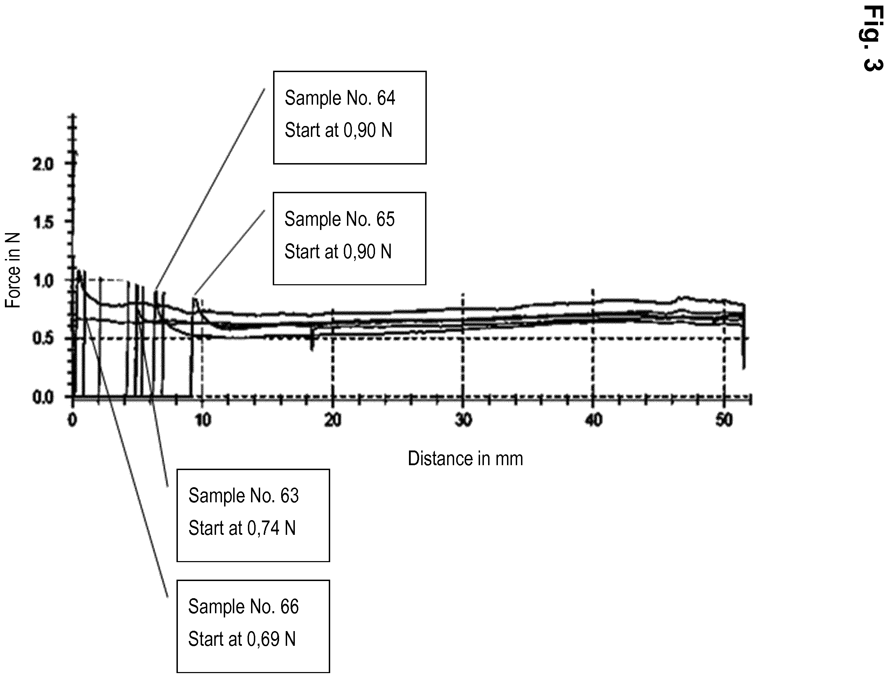

The invention is further illustrated by the examples shown in the enclosed Figures 1 to 9, wherein:
- Fig. 1: shows the piston of the invention in a side view;
- Fig. 2: shows the results of force to distance measurements of a conventional BD U 100 syringe equipped with a 30 gauge needle using water as a medium and a velocity of 60 mm/min at room temperature;
- Fig. 3: shows force to distance measurements of a syringe based on a cylinder-piston system according to the present invention equipped with a 30 gauge needle using water as a medium and a velocity of 60 mm/min at room temperature;
- Fig. 4: shows a scanning electron microscopy (SEM) image of a section of a PET cylinder injection molded at 28 °C at 25.000 fold magnification;
- Fig. 5: shows a SEM image of a section of a PET cylinder injection molded below 18 °C at 25.000 fold magnification;
- Fig. 6: shows an SEM image of an etched section of a PET cylinder injection molded at 28 °C at 10.000 fold magnification;
- Fig. 7: shows an SEM image of an etched section of a PET cylinder injection molded below 18 °C at 10.000 fold magnification;
- Fig. 8: shows an SEM image of a boundary area of an etched section of a PET cylinder injection molded at 28 °C at 150 fold magnification; and
- Fig. 9: shows an SEM image of a boundary area of an etched section of a PET cylinder injection molded below 18 °C at 150 fold magnification.

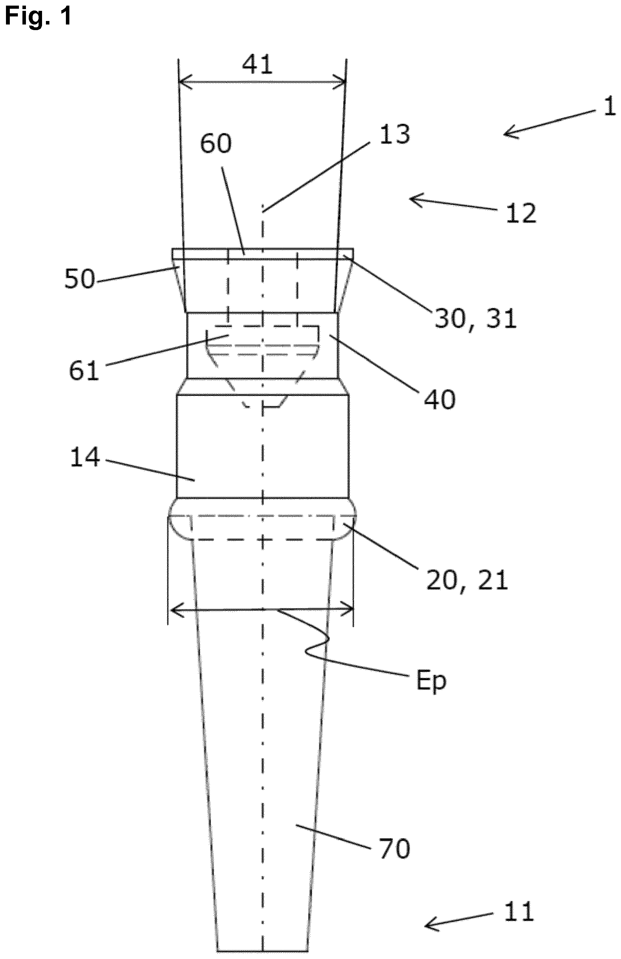

### Detailed description of the Figure 1

Fig. 1 shows a piston 1 comprising along a longitudinal axis 13 a proximal side 11 and a distal side 12, wherein on the outer surface 14 of the piston 1 at its proximal side 11 a first sealing section 20 is provided and at its distal side 12 a second sealing section 30 is provided.

In a cross section along the longitudinal axis 13 the first sealing section 20 has a convex shape 21 and the second sealing section 30 has a linear shape 31.

Between the first sealing section 20 and the second sealing section 30 the piston comprises a recess section 40 having a smaller extension 41 perpendicular to the longitudinal axis 13 than the respective extension Ep of the first sealing section 20 and the second sealing section 30, respectively.

In the transition from the second sealing section 30 to the recess section 40 the piston comprises a conical section 50.

At the rear end of the piston 1 at the distal side the piston 1 comprises an opening leading to a hollow space 61 for holding an end of a rod of the piston which is not shown here.

On the opposite end of the piston 1 at its proximal side 11 a conical protrusion 70 is provided for moving into a complementary formed space in the cylinder and removal of residues of the cylinder.

**List of reference signs**

| | |
|---|---|
| piston | 1 |
| proximal side | 11 |
| distal side | 12 |
| longitudinal axis | 13 |
| outer surface | 14 |
| | |
| first sealing section | 20 |
| convex shape | 21 |
| extension of the first sealing section | Ep |
| | |
| second sealing section | 30 |
| linear shape | 31 |
| | |
| recess section | 40 |
| extension of the recess section | 41 |
| conical section | 50 |
| opening | 60 |
| hollow space | 61 |
| conical protrusion | 70 |

## Claims

1. Cylinder-piston system comprising
a. a cylinder, wherein at least an inner section of the inner surface of the cylinder is formed from a first polymer material (C),
b. a piston (1), comprising at least one sealing section on the outer surface (14) of the piston (1), which is formed from a second polymer material (P), wherein
c. the cylinder and the piston (1) are positioned or positionable in such a way that during a sliding movement of the piston (1) with respect to the cylinder the inner section of the cylinder and the sealing section of the piston (1) slide on each other,
**characterized in that** one of said polymer materials, particularly the first polymer material (C), comprises at least a polar polymer material, particularly is selected from a polar polymer material, and the other polymer material, particularly the second polymer material (P), comprises at least a nonpolar polymer material, particularly is selected from a nonpolar polymer material.

2. Cylinder-piston system according to claim 1, **characterized in that** the complete inner surface of the cylinder, in particular the entire cylinder, is formed from said first polymer material (C).

3. Cylinder-piston system according to claim 1 or 2, **characterized in that** the complete outer surface (14) of the piston (1), in particular the entire piston (1), is formed from said second polymer material (P).

4. Cylinder-piston system according to any one of the claims 1 to 3, **characterized in that** the interface energy with respect to water of the polar polymer material is between 30 mN/m and 60 mN/m, particularly between 35 mN/m and 50 mN/m, more particularly between 40 mN/m and 45 mN/m.

5. Cylinder-piston system according to any one of the claims 1 to 4, **characterized in that** the interface energy with respect to water of the nonpolar polymer material is less than 25 mN/m, and is particularly between 10 mN/m and 20 mN/m.

6. Cylinder-piston system according to any one of the claims 1 to 5, **characterized in that** the difference of interface energies with respect to water between the first polymer material (C) and the second polymer material (P) is at least 10 mN/m, in particular at least 18 mN/m, more particularly at least 25 mN/m.

7. Cylinder-piston system according to any one of the claims 1 to 6, **characterized in that** the first polymer material (C) comprises a ball indentation hardness according to ISO 2039-1 in the range of 130 to 160 MPA, and/or the second polymer material (P) comprises a hardness of Shore A in the range of 33 to 80, in particular of 40 to 55.

8. Cylinder-piston system according to any one of the claims 1 to 7, **characterized in that** the compression set of the second polymer material (P) is in the range of 0 to 30 %, in particular in the range of in the range of 0 to 20 %, measured at 60 °C.

9. Cylinder-piston system according to any one of the claims 1 to 8, **characterized in that** the first polymer material (C) comprises a super-amorphous structure with a degree of crystallisation of less than 3 %.

10. Cylinder-piston system according to any one of the claims 1 to 9, **characterized in that** the first polymer material (C) and/or the second polymer material (P), particularly the second polymer material (C), comprises internal lubricants.

11. Cylinder-piston system according to claim 10, **characterized in that** the internal lubricants comprise, particularly are selected from at least one of the group of acid amides, more particularly primary acid amides, most particularly erucamide, oleamide, or stearamide, secondary amides, most particularly ethylene bis stearamide (EBS) or ethylene bis oleamide (EBO), acid esters, more particularly poly ethylene mono stearate (PEMS), poly ethylene di stearate (PEDS), poly ethylene tri stearate (PETS), poly ethylene co-acrylic acid stearate (PEAS), glycerol monostearate (GMS), glycerol monooleate (GMO), Montan Wax, stearyl stearate, or distearyl phthalate, fatty acids, more particularly saturated fatty acids, most particularly lauric acid, myristic acid, palmitic acid, or stearic acid, or unsaturated fatty acids, most particularly oleic acid or erucic acid, hydrocarbon waxes, more particularly polyethylene, polypropylene, OPE (Oxidized Polyethylene Wax), or paraffin, metallic soaps, more particularly calcium stearate, magnesium stearate, lead stearate, aluminium stearate, sodium stearate, tin stearate, barium stearate, cobalt stearate, or zinc stearate, or polytetrafluorethylen.

12. Cylinder-piston system according to any one of the claims 1 to 11, **characterized in that** the first polymer material (C) comprises, particularly is selected from a transparent material, in particular Polyethylenterephthalate (PET), Polybutylenterephthalate (PBT), Polymethylmethacrylate (PMMA), or polysulfone (PSU), Polycarbonate (PC), Polystyrene (PS), Cyclo-Olefin-Copolymeres (COC), Polypropylene (PP) or Polyethylene (PE), and the second polymer material (P) comprises, particularly is selected from a thermoplastic elastomer (TPE).

13. Cylinder-piston system according to any one of claims 1 to 12, **characterized in that** the first polymer material (C) comprises, particularly is selected from Polyethylenterephthalate-Glycol (PETG) or Polyethylenterephthalate-Cyclohexane Dimethanol (PET-CHDM).

14. Cylinder-piston system according to any one of claims 1 to 13, **characterized in that** the second polymer material (P) comprises, particularly is selected from a hydrogenated styrenic block copolymer, particularly styrene-ethylene/butylene-styrene (SEBS), styrene-ethylene/propylene-3-methyl-butene-styrene (SEPS), styrene-ethylene-ethylene/propylene-styrene (SEEPS), or styrene-isoprene-styrene (SIPS).

15. Cylinder-piston system according to any one of the claims 1 to 14, **characterized in that** the at least one sealing section of the piston (1) is adapted to press or presses against the inner surface of the cylinder of the cylinder-piston system, wherein the ratio (Ep/Ec) between the maximum piston extension (Ep) of the piston (1) with respect to the longitudinal axis (13) of the cylinder-piston system and the corresponding maximum cavity extension (Ec) of the cavity defined by the cylinder is 1.02 to 1.10.

16. Cylinder-piston system according to any one of the claims 1 to 15, **characterized in that** the piston (1) of the cylinder-piston-system comprises a first sealing section (20) and a second sealing section (30) as well as a recess section (40), wherein the recess section (40) is provided along the longitudinal axis (13) of the cylinder-piston-system between the first sealing section (20) and the second sealing section (30), and wherein the maximum extension (41) of the recess section (40) is smaller than the maximum extension of the first sealing section (20) and of the second sealing section (30), respectively.

17. Cylinder-piston system according to any one of the claims 1 to 16, **characterized in that**
i) the first sealing section (20) and/or the second sealing section (30) is/are an integral part of a body of the piston (1); or
ii) the first sealing section (20) and/or the second sealing section (30) is/are an additional element/additional elements provided on the body of the piston (1).

18. Cylinder-piston system according to any one of the claims 1 to 17, **characterized in that** the first sealing section (20) is provided on a proximal side (11) of the piston (1) and/or the second sealing section (30) is provided on a distal side (12) of the piston (1), wherein the cross section of the first sealing section (20) along the longitudinal axis (13) of the cylinder-piston-system has a convex shape (21) on its outer surface (14), and the cross section of second sealing section (30) along the longitudinal axis (13) of the cylinder-piston-system has a linear shape (31) on its outer surface (14).

19. Cylinder-piston system according to any one of the claims 1 to 18, **characterized in that**
i) the fill volume of the cylinder is in the range from 0.35 ml to 1.0 ml, in particular from 0.5 ml to 0.85 ml; and/or
ii) the inner diameter of the cylinder is in the range from 3 mm to 4 mm; and/or
iii) the length of the cylinder along which the piston can slide is in the range from 35 mm to 80 mm, in particular from 50 mm to 65 mm.
